# EUROPEAN PATENT SPECIFICATION

(11) **EP 3 934 568 B1**
(45) Date of publication and mention of the grant of the patent: **10.07.2024**
(21) Application number: 20705687.0
(22) Date of filing: 14.02.2020
(51) Int. Cl.: A61C 17/00, A61C 19/06

(54) **ORAL CARE APPLIANCE WITH LIGHT ILLUMINATION FOR TEETH WHITENING AND GUM HEALTH**
MUNDPFLEGEGERÄT MIT LICHTBELEUCHTUNG ZUM BLEICHEN VON ZÄHNEN UND FÜR ZAHNFLEISCHGESUNDHEIT
APPAREIL DE SOIN BUCCAL AVEC ÉCLAIRAGE DE LUMIÈRE POUR LE BLANCHIMENT DES DENTS ET LA SANTÉ DES GENCIVES

(30) Priority: 08.03.2019 US 201962815454 P
(43) Date of publication of application: 12.01.2022
(73) Proprietor: Koninklijke Philips N.V., 5656 AG Eindhoven (NL)
(72) Inventor: WANG, Tianyi, 5656 AE Eindhoven (NL)
(74) Representative: Philips Intellectual Property & Standards
(86) International application number: PCT/EP2020/053951
(87) International publication number: WO 2020/182411

(56) References cited:
- WO-A1-2013/155632
- US-A1- 2004 193 235
- US-A1- 2016 271 415
- US-A1- 2018 206 948

## Description

### Field of the Disclosure

The present disclosure is directed generally to oral care appliances for whitening teeth and increasing overall gum health

### Background

WO2013155632A1 discloses intra-oral light-therapy apparatuses.

US2004193235A1 discloses a multi-directional oral phototherapy applicator.

US2016271415A1 discloses a teeth whitening apparatus. LED light having a wavelength range of 400-500 nm that is the most suitable for the teeth whitening is irradiated to the user's teeth so that a combined sterilizing and teeth whitening function can be exhibited.

US2018206948A1 discloses a light curing dental system comprising a light emitting tray that precisely distributes an optimal amount of curing light energy to internal, external and extra oral portions of the dental arch to cure a dental material. The tray may be configured to deliver light in the 350-700 nm wavelength region for bio-stimulation of bone and soft tissue and possible reversal of osteonecrosis.

Staining and yellowing of teeth is an inevitable result of daily use. As a result, there has been an increase in consumer products that are designed to provide at-home treatments for teeth whitening. One type of at-home product emits targeted light in the "blue" wavelengths of the electromagnetic spectrum. Blue light can be used in conjunction with hydrogen peroxide (H₂O₂) to facilitate the reaction between hydrogen peroxide and staining molecules to increase the effectiveness of teeth whitening.

Additionally, light therapy or phototherapy, is a known technology that has been used by healthcare professionals to relieve pain, heal wounds, fight infections, rejuvenate aging skin, treat acne, and to improve conditions such as seasonal affective disorder (SAD). Phototherapy involves delivery of low-level "red" light at specific wavelengths to improve blood flow, increase circulation, encourage cellular turnover, and repair damaged tissue. Note that while light therapy typically involves delivery of "red" light wavelengths, and are referred to herein as "red" light, other light wavelengths, including those outside of the red or infrared spectrums, can be used, depending on the treatments to be executed, and the light wavelengths that are most effective for those treatments.

Notwithstanding available teeth whitening treatments, there is a continued need for improved systems and methods for whitening teeth and increasing overall gum health simultaneously.

### Summary of the Disclosure

The invention is defined by claim 1. The dependent claims define advantageous embodiments.

The instant disclosure is directed to an oral care appliance that produces a first wavelength of light directed to a user's teeth to facilitate teeth whitening, while simultaneously producing a second wavelength of light directed to a user's gums to promote overall gum health, providing a more complete oral healthcare within the same treatment time. The first wavelength is in the range of 400-495 nm. The second wavelength is in the range of 650-1400 nm.

These and other aspects of the various embodiments will be apparent from and elucidated with reference to the embodiment(s) described hereinafter.

### Brief Description of the Drawings

In the drawings, like reference characters generally refer to the same parts throughout the different views. Also, the drawings are not necessarily to scale, emphasis instead generally being placed upon illustrating the principles of the various embodiments.
FIG. 1 is a perspective view of an oral care appliance according to the present disclosure.
FIG. 2 is a top perspective view of an example embodiment of a plate member according to the present disclosure.
FIG. 3 is a top perspective view of an example embodiment of a plate member according to the present disclosure.
FIG. 4 is a top plan view of an oral care appliance according to the present disclosure.
FIG. 5 is a cross-sectional view of the first and second wavelengths of light directed to a user's tooth and gums.

### Detailed Description of Embodiments

The present disclosure describes various embodiments of an oral appliance for whitening teeth and increasing overall gum health. The oral care appliance has a body including a plate member and a lighting system. The plate member has a first surface, the first surface includes a top portion, a center portion, and a bottom portion. The lighting system includes a first lighting sub-system having at least one first light source, where the at least one first light source is arranged in the center portion of the plate member, and a second lighting sub-system having at least one second light source, where the at least one second light source is arranged in the top portion of the plate member and/or the bottom portion of the plate member. The at least one first light source is arranged to produce a first light at a first wavelength and the at least one second light source is arranged to produce a second light at a second wavelength.

This arrangement produces an improved overall oral healthcare experience in that the first light (e.g., "blue" light) is more efficiently focused on the user's teeth for more efficient whitening, and the second light (e.g., "near infrared" or "NIR" light) is more efficiently focused on the user's gums for more efficient absorption by the gums improving overall gum health. The specific wavelengths of light for the first and second lights do not have to be specifically blue and red or near-infrared; any wavelengths suitable to the specific treatment outcome desired can be used. These two light applications can happen simultaneously, allowing for two separate treatments within a single treatment time-frame. Furthermore, the physical separation between these light sources (e.g., D1 and/or D2 discussed in detail below), provides for a more effective and more uniform/homogenous illumination by the first and second lights on the teeth and gums, respectively. Additionally, physical separation of these sources of light may aid in heat dispersion, i.e., allowing for an enhanced control of thermal exposure to the teeth, and more importantly, to the user's gums. Furthermore, the separation allows for lower power requirements for the first and second light sources due to each source being closer to the area it is designed to effect, e.g., the teeth or the gums.

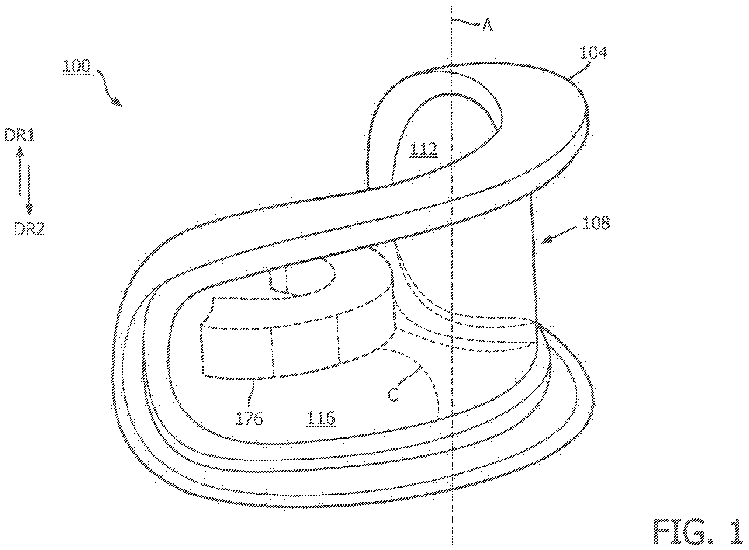

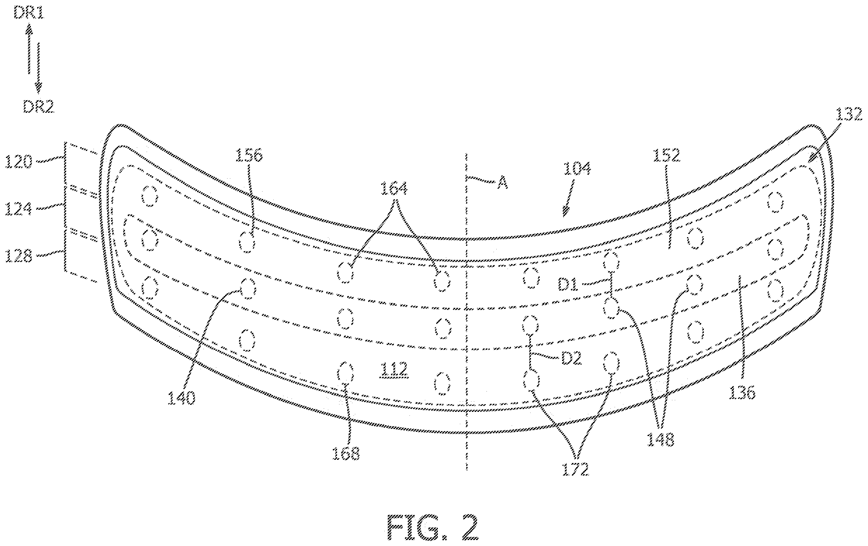

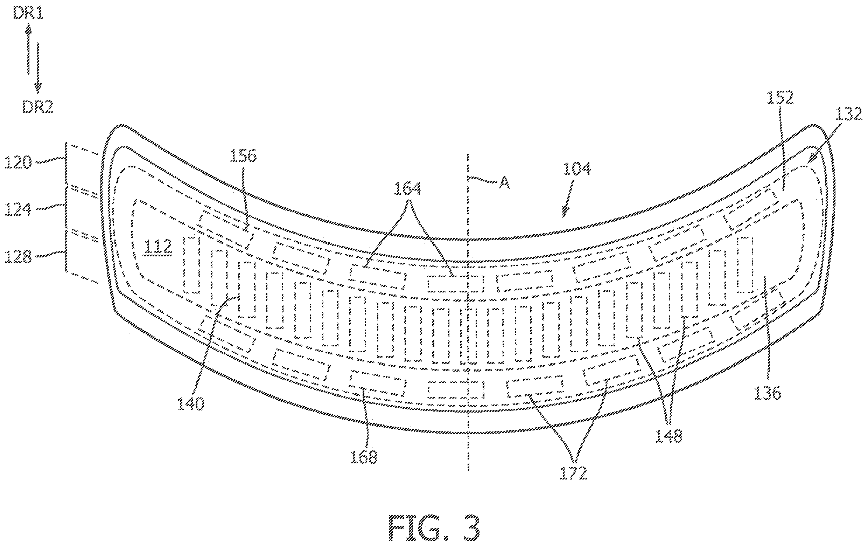

Referring to the figures, FIG. 1 illustrates a perspective view of oral care appliance 100 according to the present disclosure. Oral care appliance 100 has body 104. Body 104 includes plate member 108 which passes through an imaginary axis A, a lighting system 132 (shown in FIGS. 2 and 3), and a bite member 176. Axis A is substantially orthogonal to the surface of the ground beneath the user while using oral care appliance 100. Plate member 108 includes first surface 112 and second surface 116 substantially defining a curved plate. First surface 112 is intended to face a user's teeth T while they are engaged with bite member 176, while second surface 116 is intended to face away from the user's teeth T and away from the user when engaged with bite member 176. Plate member 108, which includes first surface 112 and second surface 116, is configured such that it forms an ergonomic, curved shape conforming substantially about a set of human teeth T. Plate member 108 can be made from materials such as, but not limited to, glass, plastics such as: Polyethylene Terephthalate, High Density Polyethylene (HDPE), Polyvinyl Chloride (PVC), Low-Density Polyethylene (LDPE), Polypropylene, Poly(methyl methacrylate)(PMMA), acrylic, or any material capable of being placed in a human mouth that at least partially reduces the attenuation of light through from first surface 112 to second surface 116 as explained in detail below. Alternatively, it should be appreciated that the material chosen for plate member 108 can be transparent or translucent.

FIG. 2 illustrates an embodiment of oral care appliance 100 and plate member 108. First surface 112 of plate member 108 is, schematically, separated into three general portions, a top portion 120, a center portion 124, and a bottom portion 128. Plate member 108 is configured such that first surface 112 is substantially planar in a vertical dimension, while being substantially curved in a horizontal dimension with a substantially constant radius of curvature. As used herein, the term "vertical dimension' is used to describe a dimension of the first surface 112 that is substantially parallel with axis A, while the term "horizontal dimension" is used to describe a dimension of the first surface 112 that is substantially orthogonal to axis A. Top portion 120 is defined as the upper most portion of first surface 112 in first direction DR1 parallel to axis A. Bottom portion 128 is defined as the lower most portion of first surface 112 in first direction DR1 parallel to axis A. Center portion 124 is defined as the portion of first surface 112 located substantially between top portion 120 and bottom portion 128 along axis A.

Lighting system 132 includes at least two lighting sub-systems, i.e., first lighting sub-system 136 and second lighting sub-system 152. First lighting sub-system 136 includes at least one first light source 140. It should be appreciated that at least one first light source 140 may be one of a plurality of first light sources 148. At least one first light source 140 is arranged to produce a first light 142 at a first wavelength of light 144. First wavelength of light 144 is intended in to be within a range of electromagnetic radiation, specifically between 400-495 nm, which substantially corresponds with "blue" light; however, a different light wavelength can be used. Second lighting sub-system 152 includes at least one second light source 156. It should be appreciated that at least one second light source 156 may be one of a plurality of second light sources 164. At least one second light source 156 is arranged to produce a second light 162 at a second wavelength of light 160. Second wavelength of light 160 is intended to be within a range of electromagnetic radiation, specifically between 650-1400 nm, which substantially corresponds with "red" or "Near Infrared" light. It can be appreciated that the second wavelength of light can with a different light wavelength than the red or infrared spectrum, depending on the desired outcome desired; however, first and second light wavelengths should differ.

Although, in a preferred embodiment, at least one first light source 140 and at least one second light source 156 are intended to be point-source light-emitting diodes (LEDs), it should be appreciated that at least one first light source 140 and at least one second light source 156 can be chosen from the group of: a light diffusing fiber (e.g., single mode, multimode, or photonic crystal fiber), an electrodeless light source, an incandescent light source, a florescent light source, a hollow-cathode light source, a sulfur light source, a neon and argon light source, a laser light source, and a plasma light source.

Furthermore, in some embodiments lighting system 132 includes at least one third light source 168. At least one third light source 168 can be a part of second lighting sub-system 152, or be a part of a separate third lighting sub-system (not shown). The at least one third light source 168 is arranged to produce the second light 162 at the second wavelength of light 160, i.e., light substantially in the "red" or "near infrared" spectrums. It should be appreciated that at least one third light source may be one of a plurality of third light sources 172.

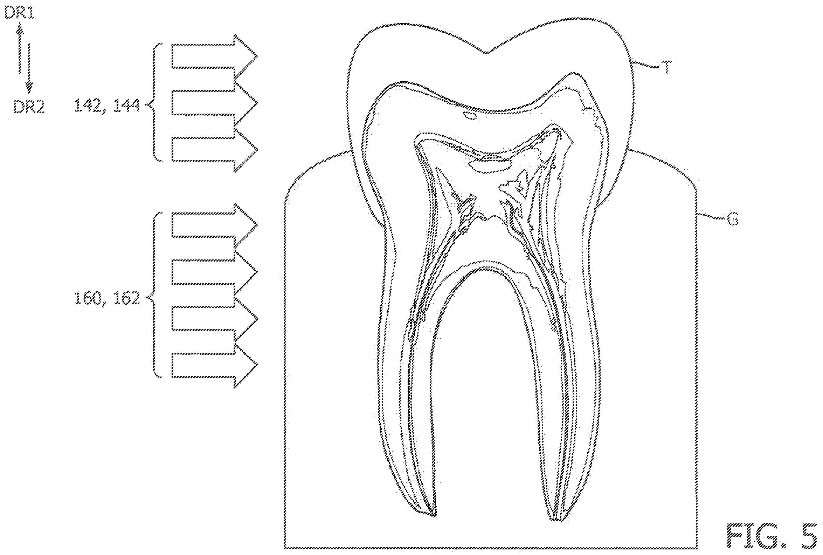

The at least one first light source 140 is separated from the at least one second light source 156 a first vertical distance D1 along axis A in direction DR1, such that a substantial quantity of the first wavelength of light 144 emitted by the at least one first light source 140 is directed to the user's teeth T (shown in FIG. 5), while a substantially quantity of the second wavelength of light 160 emitted by the at least one second light source 156 is directed to the user's gums G (shown in FIG. 5). Additionally, at least one third light source 168 is separated from at least one first light source 140 by a second vertical distance D2 along axis A, where vertical distance D2 is substantially equal to distance D1, in second direction DR2, opposite first direction DR1, such that a substantial quantity of the second wavelength of light 160 emitted by the at least one third light source 168 is directed to the user's gums G (shown in FIG. 5).

FIG. 3 illustrates an embodiment of oral care appliance 100 having plate member 108. Here, first surface 112 contains at least one first light source 140, at least one second light source 156, and at least one third light source 168 within center portion 124, top portion 120, and bottom portion 128, respectively, where the at least one first, second, and third light sources are arrays of LEDs instead of point-source LEDs shown in FIG. 2.

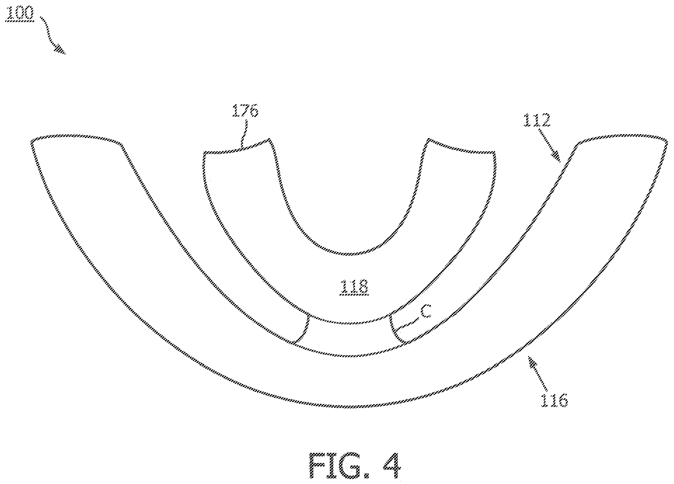

FIG. 4 illustrates a top plan view of the oral care appliance 100 shown in FIG. 1. In this view, bite member 176 and bite surface 180 can be seen. During use, the user places bite member within the user's mouth and provides a biting force on at least bite surface 180. In one example embodiment, bite member 176 is configured such that it substantially forms a negative of the user's mouth, similar to a various known mouth guards used for sporting events, with the exception of a front wall that would normally protect a user's teeth from contact. Without this front wall, bite member 176 allows for the light produced by at least one first light source 140, at least one second light source 156, and at least one third light source 168 to reach the user's teeth and gums without obstruction. Additionally, plate member 108 is configured such that during operation, the user's lips would encompass plate member 108 leaving a direct, unobstructed, path for the light produced by the at least one first light source 140, at least one second light source 156, and at least one third light source 168 to contact the user's teeth and gums. Bite member 176 is engaged with plate member 108 by a connector C. Although connector C is illustrated as being integral with plate member 108 and bite member 176, it should be appreciated that connector C could be removably engaged with plate member 108.

FIG. 5 illustrates a cross-sectional view of a user's teeth T and gums G. In this view, the effect of the vertical separation of at least one first light source 140 and at least one second light source 156 can be seen. The separation these light sources first distance D1 creates a more efficient concentration of blue light directed at the user's teeth T, as well as a more efficient concentration of red or near infrared light directed at the user's gums G. It should be appreciated that although only one tooth and gum section is shown, this concept applies to the plurality of user's teeth T throughout the top and bottom rows of teeth within the user's mouth.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

It should be appreciated that the term "substantially" is synonymous with terms such as "nearly," "very nearly," "about," "approximately," "around," "bordering on," "close to," "essentially," "in the neighborhood of," "in the vicinity of," etc., and such terms may be used interchangeably as appearing in the specification and claims. It should be appreciated that the term "proximate" is synonymous with terms such as "nearby," "close," "adjacent," "neighboring," "immediate," "adjoining," etc., and such terms may be used interchangeably as appearing in the specification and claims. The term "approximately" is intended to mean values within ten percent of the specified value.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of'.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "having," "containing," "involving," and the like are to be understood to be open-ended, i.e., to mean including but not limited to.

## Claims

1. An oral care appliance (100) comprising:
a body (104), the body comprising:
a plate member (108) having a first surface (112), wherein the first surface of the plate member is a curved surface comprising:
a top portion (120);
a center portion (124); and,
a bottom portion (128); and,
a lighting system (132) comprising:
a first lighting sub-system (136) having at least one first light source (140), the at least one first light source arranged in the center portion of the plate member;
a second lighting sub-system (152) having at least one second light source (156), the at least one second light source arranged in the top portion of the plate member or the bottom portion of the plate member;
wherein the at least one first light source is arranged to produce a first light (142) at a first wavelength (144) and the at least one second light source is arranged to produce a second light (162) at a second wavelength (160); **characterized in that**
the first wavelength is in the range of 400-495 nm; and
the second wavelength is in the range of 650-1400 nm.

2. The oral care appliance of claim 1, wherein the body further comprises a bite member (176) having a bite surface (180) arranged to contact a user's teeth (T).

3. The oral care appliance of claim 1, wherein the at least one first light source is separated from the at least one second light source by a first vertical distance (D1).

4. The oral care appliance of claim 1, wherein at least one of the at least one first light source and the at least one second light source is a light-emitting diode (LED).

5. The oral care appliance of claim 1, wherein the at least one first light source and the at least one second light source are selected from the group of: a light diffusing fiber, an electrodeless light source, an incandescent light source, a florescent light source, a hollow-cathode light source, a sulfur light source, a neon and argon light source, a laser light source, and a plasma light source.

6. The oral care appliance of claim 1, wherein the second lighting sub-system (152) further comprises at least one third light source (168), the at least one second light source arranged in the top portion of the plate member and the at least one third light source arranged in the bottom portion of the plate member;
wherein the at least one third light source is arranged to produce a second light (162) at a second wavelength (160).

7. The oral care appliance of claim 6, wherein the at least one third light source and the at least one first light source are separated by a second vertical distance (D2).

## Patentansprüche

1. Mundpflegegerät (100), umfassend:
einen Körper (104), wobei der Körper umfasst:
ein Plattenelement (108) mit einer ersten Oberfläche (112), wobei die erste Oberfläche des Plattenelements eine gekrümmte Oberfläche ist, umfassend:
einen oberen Abschnitt (120);
einen mittleren Abschnitt (124); und
einen unteren Abschnitt (128); und
ein Beleuchtungssystem (132), umfassend:
ein erstes Beleuchtungsteilsystem (136) mit zumindest einer ersten Lichtquelle (140), wobei die zumindest eine erste Lichtquelle in dem mittleren Abschnitt des Plattenelements angeordnet ist;
ein zweites Beleuchtungsteilsystem (152) mit zumindest einer zweiten Lichtquelle (156), wobei die zumindest eine zweite Lichtquelle in dem oberen Abschnitt des Plattenelements oder in dem unteren Abschnitt des Plattenelements angeordnet ist;
wobei die zumindest eine erste Lichtquelle so angeordnet ist, dass sie ein erstes Licht (142) mit einer ersten Wellenlänge (144) erzeugt, und die zumindest eine zweite Lichtquelle so angeordnet ist, dass sie ein zweites Licht (162) mit einer zweiten Wellenlänge (160) erzeugt; **dadurch gekennzeichnet, dass**
die erste Wellenlänge in dem Bereich von 400-495 nm liegt; und
die zweite Wellenlänge in dem Bereich von 650-1400 nm liegt.

2. Mundpflegegerät nach Anspruch 1, wobei der Körper weiter ein Beißelement (176) mit einer Beißfläche (180) umfasst, welche zum Kontakt mit den Zähnen (T) eines Benutzers angeordnet ist.

3. Mundpflegegerät nach Anspruch 1, wobei die zumindest eine erste Lichtquelle durch einen ersten vertikalen Abstand (D1) von der zumindest einen zweiten Lichtquelle getrennt ist.

4. Mundpflegegerät nach Anspruch 1, wobei zumindest eine der zumindest einen ersten Lichtquelle und der zumindest einen zweiten Lichtquelle eine Leuchtdiode (LED) ist.

5. Mundpflegegerät nach Anspruch 1, wobei die zumindest eine erste Lichtquelle und die zumindest eine zweite Lichtquelle aus der folgenden Gruppe ausgewählt sind: eine lichtstreuende Faser, eine elektrodenlose Lichtquelle, eine Glühlichtquelle, eine Leuchtstofflampe, eine Hohlkathodenlichtquelle, eine Schwefellichtquelle, eine Neon- und Argonlichtquelle, eine Laserlichtquelle und eine Plasmalichtquelle.

6. Mundpflegegerät nach Anspruch 1, wobei das zweite Beleuchtungsteilsystem (152) weiter zumindest eine dritte Lichtquelle (168) umfasst, wobei die zumindest eine zweite Lichtquelle in dem oberen Abschnitt des Plattenelements angeordnet ist und die zumindest eine dritte Lichtquelle in dem unteren Abschnitt des Plattenelements angeordnet ist;
wobei die zumindest eine dritte Lichtquelle dazu angeordnet ist, ein zweites Licht (162) mit einer zweiten Wellenlänge (160) zu erzeugen.

7. Mundpflegegerät nach Anspruch 6, wobei die zumindest eine dritte Lichtquelle und die zumindest eine erste Lichtquelle durch einen zweiten vertikalen Abstand (D2) getrennt sind.

## Revendications

1. Appareil de soins buccaux (100) comprenant :
un corps (104), le corps comprenant :
un élément plaque (108) présentant une première surface (112), dans lequel la première surface de l'élément plaque est une surface incurvée comprenant :
une partie supérieure (120) ;
une partie centrale (124) ; et,
une partie inférieure (128) ; et,
un système d'éclairage (132) comprenant :
un premier sous-système d'éclairage (136) présentant au moins une première source de lumière (140), la au moins une première source de lumière étant agencée dans la partie centrale de l'élément plaque ;
un second sous-système d'éclairage (152) présentant au moins une deuxième source de lumière (156), la au moins une deuxième source de lumière étant agencée dans la partie supérieure de l'élément plaque ou dans la partie inférieure de l'élément plaque ;
dans lequel la au moins une première source de lumière est agencée pour produire une première lumière (142) à une première longueur d'onde (144) et la au moins une deuxième source de lumière est agencée pour produire une seconde lumière (162) à une seconde longueur d'onde (160) ; **caractérisé en ce que**
la première longueur d'onde est dans la plage de 400-495 nm ; et
la seconde longueur d'onde est dans la plage de 650-1400 nm.

2. Appareil de soins buccaux selon la revendication 1, dans lequel le corps comprend en outre un élément à mordre (176) présentant une surface à mordre (180) agencée pour venir en contact avec les dents (T) d'un utilisateur.

3. Appareil de soins buccaux selon la revendication 1, dans lequel la au moins une première source de lumière est séparée de la au moins une deuxième source de lumière d'une première distance verticale (D1).

4. Appareil de soins buccaux selon la revendication 1, dans lequel au moins l'une de la au moins une première source de lumière et de la au moins une deuxième source de lumière est une diode électroluminescente (DEL).

5. Appareil de soins buccaux selon la revendication 1, dans lequel la au moins une première source de lumière et la au moins une deuxième source de lumière sont sélectionnées dans le groupe comprenant : une fibre diffusant la lumière, une source de lumière sans électrode, une source de lumière incandescente, une source de lumière fluorescente, une source de lumière à cathode creuse, une source de lumière au soufre, une source de lumière au néon et à l'argon, une source de lumière laser et une source de lumière à plasma.

6. Appareil de soins buccaux selon la revendication 1, dans lequel le second sous-système d'éclairage (152) comprend en outre au moins une troisième source de lumière (168), la au moins une deuxième source de lumière agencée dans la partie supérieure de l'élément plaque et la au moins une troisième source de lumière agencée dans la partie inférieure de l'élément plaque ;
dans lequel la au moins une troisième source de lumière est agencée pour produire une seconde lumière (162) à une seconde longueur d'onde (160).

7. Appareil de soins buccaux selon la revendication 6, dans lequel la au moins une troisième source de lumière et la au moins une première source de lumière sont séparées d'une seconde distance verticale (D2).
